# FASCICULE DE BREVET EUROPEEN

(11) **EP 2 844 734 B1**
(45) Date de publication et mention de la délivrance du brevet: **18.10.2017**
(21) Numéro de dépôt: 13715327.6
(22) Date de dépôt: 15.03.2013
(51) Int. Cl.: C12N 1/12, C12N 13/00, C12P 7/64, C12P 23/00, C12R 1/89

(54) **PRODUCTION DE LUTÉINE EN MODE MIXOTROPHE PAR SCENEDESMUS**
HERSTELLUNG VON LUTEIN IN MIXOTROPHEM MODUS DURCH SCENEDESMUS
PRODUCTION OF LUTEIN IN MIXOTROPHIC MODE BY SCENEDESMUS

(30) Priorité: 16.03.2012 FR 1252381
(43) Date de publication de la demande: 11.03.2015
(73) Titulaire: Fermentalg, 33500 Libourne (FR)
(72) Inventeur: ROMARI, Khadidja, 34200 Sète (FR); GODART, François, 33870 Vayres (FR); CALLEJA, Pierre, 33500 Libourne (FR)
(74) Mandataire: Be IP
(86) Numéro de dépôt international: PCT/FR2013/050544
(87) Numéro de publication internationale: WO 2013/136027

(56) Documents cités:
- WO-A1-96/21723
- WO-A1-2009/134114
- HONG-WEI YEN ET AL: "The Comparison of Lutein Production by Scenesdesmus sp. in the Autotrophic and the Mixotrophic Cultivation", APPLIED BIOCHEMISTRY AND BIOTECHNOLOGY, vol. 164, no. 3, 4 décembre 2010 (2010-12-04), pages 353-361, XP055047383, ISSN: 0273-2289, DOI: 10.1007/s12010-010-9139-1
- SÁNCHEZ J F ET AL: "Biomass and lutein productivity of Scenedesmus almeriensis: influence of irradiance, dilution rate and temperature", APPLIED MICROBIOLOGY AND BIOTECHNOLOGY, SPRINGER, BERLIN, DE, vol. 79, no. 5, 20 mai 2008 (2008-05-20), pages 719-729, XP019623647, ISSN: 1432-0614

## Description

L'invention se rapporte à un procédé de culture en mode mixotrophe, en présence d'un apport variable et/ou discontinu de lumière, notamment sous forme de flashs, d'une microalgue du genre *Scenedesmus.* Le procédé permet d'obtenir un haut rendement en biomasse et un enrichissement des microalgues ainsi cultivées en lutéine. L'invention se rapporte aussi à une nouvelle souche de microalgue appartenant au genre *Scenedesmus,* particulièrement adaptée à la production de caroténoïdes. Cette nouvelle souche de *Scenedesmus* sp. est utile pour produire de la lutéine en mode mixotrophe.

### Préambule

Les microalgues sont des microorganismes photosynthétiques à caractère autotrophe, c'est-à-dire ayant l'aptitude de croître de manière autonome par photosynthèse.

Les microalgues se développent aussi bien dans les milieux aquatiques marins qu'en eaux douces ou saumâtres, ainsi que dans divers habitats terrestres.

La plupart des espèces de microalgues rencontrées dans l'eau douce ou les océans sont généralement autotrophes, c'est-à-dire qu'elles ne peuvent croître que par photosynthèse. Pour celles-ci, la présence dans leur milieu de substrats carbonés ou de matière organique ne leur est pas favorable et n'améliore pas leur croissance. Cependant, un certain nombre d'espèces de microalgues, de familles et d'origines très diverses, s'avèrent ne pas être généralement autotrophes. C'est ainsi que certaines d'entre elles, dites hétérotrophes, sont capables de se développer en l'absence totale de lumière, par fermentation, c'est-à-dire en exploitant la matière organique.

D'autres espèces de microalgues, pour lesquelles la photosynthèse reste indispensable à leur développement, sont capables à la fois de tirer parti de la photosynthèse et de la matière organique présente dans leur milieu. Ces espèces intermédiaires, dites mixotrophes, peuvent être cultivées à la fois en présence de lumière et de matière organique.

Cette particularité des algues dites « mixotrophes » semble être liée à leur métabolisme, qui leur permet d'opérer simultanément photosynthèse et fermentation. Les deux types de métabolisme coexistent avec un effet global positif sur la croissance des algues [Yang, C. et al. (2000) ; Biochemical Engineering Journal, 6:87-102].

Les microalgues font l'objet actuellement de nombreux projets industriels car certaines espèces sont capables d'accumuler ou de secréter des quantités importantes de caroténoïdes. Les caroténoïdes, dont environ 600 sont connus, sont des pigments plutôt orange et jaunes répandus chez de très nombreux organismes vivants. Liposolubles, ils sont en général facilement assimilables par les organismes. Ils appartiennent à la famille chimique des terpénoïdes, formés à partir de la polymérisation d'unités isoprènes à structure aliphatique ou alicyclique. On regroupe sous le terme de caroténoïde les carotènes et les xanthophylles.

Les caroténoïdes ont plusieurs fonctions chez les microalgues. Ils sont en effet non seulement impliqués dans un processus d'absorption de lumière, mais ils contribuent également à la fois à la stabilisation de la structure des complexes photosynthétiques et favorisent leur fonction, ainsi qu'au piégeage des dérivés actifs de l'oxygène et à la dissipation de l'excès d'énergie. L'activité intrinsèque anti-oxydante des caroténoïdes constitue la base de leur action protectrice contre le stress oxydatif.

Les propriétés anti-oxydantes de ces molécules sont en effet très intéressantes pour l'industrie alimentaire et l'industrie pharmaceutique. L'adjonction d'antioxydants à des produits alimentaires peut inhiber ou ralentir l'oxydation par des radicaux libres, ainsi qu'interrompre la propagation de ces chaines de radicaux libres.

Les caroténoïdes sont utilisés en tant que pigments, mais ils ont aussi un rôle important pour la santé de l'homme en tant qu'agents antioxydants. Ils ont également la capacité de stimuler le système immunitaire.

La lutéine (du latin *luteus,* jaune) (C₄₀H₅₆O₂) est une xanthophylle, trouvée dans le jaune d'oeuf, les légumes jaunes (maïs, carotte) ou à feuilles vertes (épinards, oseille) et des fleurs comestibles telles que l'oeillet d'Inde (Tagetes).

A l'heure actuelle, cette molécule est utilisée principalement en tant qu'agent de coloration dans l'industrie alimentaire. Cependant, l'on constate son utilisation croissante en tant que complément alimentaire. La lutéine, ainsi que la zéaxanthine en quantités beaucoup moins importantes, sont les seuls caroténoïdes qui sont absorbés dans le sang après ingestion et accumulés dans la rétine humaine. La lutéine est associée à une possible réduction des risques liés aux lésions oculaires et cutanées causées par la lumière bleue. Elle est notamment associée à la prévention de la dégénérescence maculaire liée à l'âge (DMLA).

Actuellement, la lutéine est obtenue à partir de pétales de calendula après un procédé d'extraction qui donne des oléorésines à des concentrations en lutéine qui varient entre 5 et 50 %, la plupart sous la forme diester. La lutéine ainsi obtenue peut être encore purifiée par saponification, concentration et recristallisation finale pour donner sa forme cristalline. La lutéine sous forme cristalline est néanmoins difficile à manipuler et est vendue en suspension dans l'huile de maïs ou de tournesol.

Cependant, le calendula présente des inconvénients comme source de lutéine. Les fleurs doivent être récoltées périodiquement et les pétales doivent être séparés avant l'extraction. Le contenu de lutéine dans les pétales est variable et peut descendre jusqu'à 0,03 %. Le résultat est que la production des pétales de calendula est un procédé demandant une main d'oeuvre intensive et de grandes surfaces de production.

La production de lutéine par voie chimique synthétique est beaucoup plus chère que la voie d'extraction des calendulas. Les autres sources de lutéine existantes (crustacés, jaunes d'oeuf) ont une disponibilité limitée ou ont un contenu assez faible (maïs) pour rendre ces sources rentables pour la production de lutéine à l'échelle industrielle.

La production de la lutéine par des microalgues représente une alternative avantageuse par rapport à la production par la voie chimique ainsi que par la voie végétale. Elle demande moins de main d'oeuvre par rapport à la voie végétale et est beaucoup plus rentable par rapport à la voie chimique.

De nouvelles sources de caroténoïdes, notamment de lutéine, doivent donc être recherchées afin de répondre, dans le futur, à la demande croissante du marché pour ces molécules.

A l'heure actuelle, la classification des algues se fonde encore largement sur des critères morphologiques et sur la nature des pigments photosynthétiques que contiennent leurs cellules. De ce fait, elle est peu indicative du caractère autotrophe, hétérotrophe ou mixotrophe des espèces d'algues, alors que ces dernières recouvrent une très grande diversité d'espèces et de formes [Dubinsky et al. (2010); Hydrobiologia, 639:153-171].

La classification taxonomique des algues eucaryotes contient 14 phylums. Parmi des espèces des différentes classes composant ces phylums, qui produisent des acides gras, il existe des variations importantes en ce qui concerne la teneur des microalgues en caroténoïdes. Par ailleurs, les proportions relatives des différentes caroténoïdes, notamment de lutéine, dans les profils lipidiques, varient selon l'espèce et les conditions de culture.

Pour mettre en oeuvre la production des acides gras par des microalgues à l'échelle industrielle, plusieurs facteurs doivent être pris en compte. Par exemple, les cultures peuvent être réalisées en condition autotrophe, mixotrophe ou hétérotrophe selon la souche, la température, les conditions de lumière et la taille des fermenteurs. Par exemple, les cultures peuvent également être réalisées dans des conteneurs d'un litre, dans un laboratoire, dans des photobioréacteurs, et dans des conteneurs de 100 000 litres ou bien dans des bassins ouverts (plusieurs hectares). Toutefois, les dépenses énergétiques et autres ressources telles que la main d'oeuvre et la facilité de poursuivre la culture doivent être prises en compte en développant des conditions de culture idéales.

En tout état de cause, il est souhaitable que les microalgues soient cultivées dans des conditions optimales pour augmenter le rendement en caroténoïdes à produire. Ainsi, il est préférable d'avoir un rendement le plus élevé possible (par exemple au-delà de 30 g de matière sèche par litre de culture et plus de 0,9 % de caroténoïdes par rapport à la matière sèche).

Parmi les différentes microalgues capables de produire de la lutéine, uniquement *Murielopsis* spp. et *Scenedesmus almeriensis* ont été testées dans des conditions de culture appropriées pour la production de la lutéine à grande échelle.

*Scenedesmus* est une microalgue verte d'eau douce appartenant à l'ordre des *Chloroccocales* et à la famille des *Scénédesmacées. Scenedesmus* se présente sous la forme de cénobes renfermant 2, 4 ou 8 cellules linéaires ou rangées alternativement. Le nombre de cellules dépend des conditions de vie de l'organisme. Les cellules sont de forme ellipsoïdale ou fusiforme, chacune présente un plaste portant d'ordinaire un pyrénoïde bien visible. Elles ont une largeur de 2,2 à 9,6 µm et une longueur de 6 à 15 µm. La multiplication se fait par autosporulation via le fractionnement des parois latérales des cellules : chaque cellule redonne un cénobe complet [Bourelly, P. (1966) Les algues d'eau douce. Initiation à la systématique. Tome I : Les Algues vertes].

Dans le brevet américain US 8 067 225, une culture de *Scenedesmus almeriensis* en autotrophie dans le réacteur tubulaire de 4000 litres a produit une biomasse avec un contenu de lutéine pouvant aller jusqu'à 0,5 % de la biomasse sèche. Les auteurs de ce document ont trouvé qu'une température de 30°C et un pH à 8, sans l'addition de vitamines, étaient les conditions les plus appropriées pour la croissance de *Scenedesmus almeriensis.*

Dans la demande de brevet internationale WO 2010/063256, des souches de *Scenedesmus* cultivées donnaient un rendement en lutéine de 2 à 6 mg/g (0,6 %) de matière sèche. Les cultures ont été effectuées en conditions autotrophes, dans un milieu minéral comprenant des phosphates et des nitrates en quantités limitées, à une température comprise entre 16 et 35 °C et à un pH compris entre 6 et 8.

Dans l'article *"*Effect of acetate on growth and ammonium uptake in the microalga Scenedesmus obliquus", publié dans Physiologia Plantarum 1994, vol. 91, no.4, pp. 729-734, la souche *Scenedesmus obliquus* a été testée en conditions de culture différentes. En conditions mixotrophes, le taux de croissance des cellules a été plus important que celui respectivement obtenu en conditions de culture autotrophe ou hétérotrophe (qui ont donné des résultats similaires), ce qui contraste avec ce que l'on avait observé avec S. *falcatus,* qui est capable d'utiliser l'acétate en conditions lumineuses, mais est incapable de croître en conditions hétérotrophes [Fingergut, U., Groeneweg, J. & Soeder, C.J., Acetate utilization in Scenedesmus falcatus, an alga from high-rate ponds (1990), Algol. Stud. 60 :57-64].

Yen et al. (Applied Biochemistry and Biotechnology 2010, 164(3):353-361) décrit la culture autotrophique et mixotrophique de Scenedesmus et la production de lutéine lors de cette culture. Il serait souhaitable de pouvoir obtenir des rendements en lutéine plus importants pour une exploitation industrielle plus efficace et rentable.

Ainsi, c'est au terme de nombreuses expérimentations dans des conditions de lumière inhabituelles et par l'adjonction de différents substrats que le demandeur est parvenu à isoler des souches de microalgue du genre *Scenedesmus* cultivables en mode mixotrophe, permettant, dans les conditions de la présente invention, une production à haut rendement de caroténoïdes, notamment de lutéine.

Une souche (FCC 174) représentative des nouvelles souches de *Scenedesmus* sp. ainsi isolées et sélectionnées, a été déposée auprès de la CCAP (Culture Collection of Algae and Protozoa, Scottish Association for Marine Science, Dunstaffnage Marine Laboratory, Oban, Argyll PA371QA, Ecosse, Royaume-Uni) selon les dispositions du Traité de Budapest, le 8 mars 2012 sous le numéro d'accession CCAP 276/75.

Le procédé de culture et de sélection a consisté plus particulièrement à cultiver les microalgues en conditions de mixotrophie, en présence d'un éclairement variable et/ou discontinu, notamment sous forme de flashs, avec une gamme de variations d'intensité lumineuse et une fréquence spécifiques.

L'alternance rapprochée de phases éclairées et de phases obscures ou de moindre intensité lumineuse, perçue généralement comme stressante par les microalgues, a permis, de façon surprenante, d'obtenir des souches de Scenedesmus sp. Une production élevée de biomasse, de caroténoïdes et plus particulièrement de la lutéine. Cette mise en oeuvre des souches selon l'invention ouvre la perspective d'une production industrielle de la lutéine, dans des fermenteurs bénéficiant d'un apport lumineux réduit, et devrait donc permettre de réaliser des économies d'énergie par rapport aux modes de culture autotrophes.

Les différents aspects et avantages de l'invention sont détaillés ci-après.

### Description détaillée

La présente invention a donc pour objet un procédé de culture des microalgues du genre Scenedesmus, en mode mixotrophe, dans des conditions d'éclairement discontinu. L'éclairement présente des variations d'intensité, entre les phases éclairées et les phases obscures, dont l'amplitude est comprise entre 50 µmol. m-2. s-1 et 1000 µmol. m-2. s-1, de préférence entre 50 et 400 µmol. m-2. s-1. Ces variations peuvent généralement avoir lieu entre 2 et 3600 fois par heure, de préférence entre 2 et 200 fois par heure. Ces conditions de culture permettent d'apporter une quantité définie de lumière. Cet apport lumineux peut comporter des phases d'éclairement discontinu pouvant avoir des amplitudes identiques ou différentes. L'éclairement peut être notamment sous forme de flashs.

Ce procédé a pour avantage d'augmenter le rendement en biomasse obtenu de la culture. Il a aussi pour avantage d'enrichir les microalgues ainsi cultivées en lutéine. Ce procédé peut être également utilisé pour sélectionner des souches du genre *Scenedesmus,* à caractère mixotrophe, et ayant un haut rendement en caroténoïdes, notamment de la lutéine.

La culture en mode mixotrophe de cette microalgue s'effectue préférentiellement en présence de 5 mM à 1 M, de préférence de 50 mM à 800 mM, plus préférentiellement de 70 mM à 600 mM, et encore plus préférentiellement de 100 mM à 500 mM d'un substrat carboné organique. L'apport du substrat est assuré continuellement pendant la culture, afin de permettre aux cellules d'accumuler une concentration importante de caroténoïdes. Du substrat additionnel est ajouté au milieu de culture pendant le procédé de culture pour maintenir une concentration constante. Ce substrat carboné organique comprend préférentiellement, sous forme pure ou en mélange : du glucose, des dérivés de cellulose, de lactate, de l'amidon, du lactose, du saccharose, de l'acétate et/ou du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés adaptés à la culture en mixotrophie des microalgues selon l'invention.

Ce procédé est plus particulièrement destiné à la mise en oeuvre de nouvelles souches de microalgues du genre *Scenedesmus* (Phylum: *Chlorophyta,* Ordre: *Chlorococcales* Famille: *Scenedesmaceae)* [ITIS Catalogue of Life, 2010] sélectionnées pour leur caractère mixotrophe, notamment pour leur capacité à être cultivées avec un apport lumineux supérieur à 10 µE, dans un milieu minéral, par exemple le milieu BG11 [Rippka et al. (1979) Rippka, R., J. Deruelles, J. Waterbury, M. Herdman and R. Stanier, Generic assignments, strain histories and properties of pure cultures of cyanobacteria. J.Gen. Microbiol. 111: 1-61] dans lequel est ajouté un substrat carboné organique. De préférence, le substrat carboné organique comprend du glucose, du saccharose, dans une concentration équivalente ou supérieure à 5 mM.

Ces nouvelles souches de *Scenedesmus* sp. peuvent être isolées et sélectionnées selon le procédé de sélection et de culture décrit plus loin.

Une souche représentative des souches de *Scenedesmus* sp. est la souche FCC 174 isolée par le demandeur et déposée à la CCAP, le 8 mars 2012, sous le numéro CCAP 276/75. De telles souches sont capables de produire des quantités significatives de biomasse ainsi que de la lutéine quand elles sont cultivées en mode mixotrophe avec un apport en lumière discontinu, selon l'invention.

Les souches de *Scenedesmus* isolées permettent de produire généralement, en conditions de mixotrophie selon l'invention, des quantités significatives de biomasse d'environ 30-50g/l, généralement d'environ 35-40g/l. Elles permettent également d'obtenir ainsi des lipides riches en lutéine, ladite lutéine pouvant représenter plus de 1% en poids par rapport au poids de la matière sèche. Elles permettent en outre d'obtenir un rendement en lutéine de plus de 10 %, plus de 25%, ou encore plus de 50%, de matière hydrophobe totale contenue dans les microalgues. La matière hydrophobe de la microalgue comporte des lipides et des caroténoïdes.

Dans la présente invention, la productivité en lutéine obtenue avec la souche FCC 174, isolée par le demandeur, d'une culture en conditions mixotrophes en présence d'un éclairement variable et/ou discontinu, notamment sous forme de flashs, peut représenter plus de 2 fois, généralement plus de 5 fois la productivité d'une culture avec la même souche effectuée en mode hétérotrophe. Par mode hétérotrophe, on entend des conditions de culture avec un milieu de culture identique, mais sans apport de lumière.

La biomasse obtenue avec la souche FCC 174, isolée par le demandeur, d'une culture en conditions mixotrophes en présence d'un éclairement variable et/ou discontinu, notamment sous forme de flashs, est généralement 50 à 100 fois, généralement 80 fois supérieur à celle d'une culture avec la même souche effectuée en mode autotrophe.

L'invention a ainsi pour objet un procédé de culture de microalgues du genre *Scenedesmus,* en mode mixotrophe, en présence d'un éclairement variable et/ou discontinu au cours du temps, par exemple, sous forme de flashs, notamment en vue de produire de la lutéine.

Il est apparu qu'un éclairement variable et/ou discontinu des cultures, en particulier dans la mise en oeuvre d'une culture en mode mixotrophe, avait un impact favorable sur le développement des algues et permettait d'accroître la productivité de celles-ci, notamment en ce qui concerne leur production de matière hydrophobe. Sans être lié par la théorie, l'inventeur estime qu'un apport discontinu et/ou variable de lumière aux microalgues a pour effet de provoquer un « stress » favorable à la croissance et à la synthèse des caroténoïdes. Ce phénomène pourrait s'expliquer, en partie, par le fait que dans la nature, les microalgues ont tendance à accumuler des réserves de matière hydrophobe pour résister aux contraintes de leur environnement.

Par éclairement discontinu, il faut entendre un éclairement ponctué par des périodes d'obscurité. Les périodes d'obscurité peuvent occuper plus d'un quart du temps, de préférence la moitié du temps ou plus, durant lequel les algues sont cultivées.

Selon un aspect préféré de l'invention, l'éclairement est discontinu et plus préférentiellement sous forme de flashs. Un flash, au sens de l'invention, est un éclairement lumineux de courte durée, c'est-à-dire de moins de 30 minutes. La durée du flash peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être de 1/10 d'une seconde, ou de 2/10 d'une seconde, ou de 3/10 d'une seconde, ou de 4/10 d'une seconde ou de 5/10 d'une seconde, ou de 6/10 d'une seconde, ou de 7/10 d'une seconde, ou de 8/10 d'une seconde, ou de 9/10 d'une seconde. L'éclairement lumineux, ou le flash, est généralement d'une durée supérieure à 15 secondes. La durée du flash est généralement comprise entre 5 secondes et 10 minutes, de préférence entre 10 secondes et 2 minutes, plus préférentiellement entre 20 secondes et 1 minute.

En général, le nombre de flashs est compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre, 2 et 200 préférentiellement entre 10 et 150, plus préférentiellement entre 15 et 100, et plus préférentiellement encore entre 20 et 50 par heure. Les flashs peuvent être émis à intervalle régulier ou non dans le temps. En cas d'émission à intervalle régulier, le nombre de flashs par heure correspond alors à une fréquence (F) ayant une période temporelle (T), étant considéré que F = 1/T. Cette période temporelle peut être comprise entre 1 seconde et 30 minutes, ou entre 1 seconde et 36 secondes, ou encore entre 1,2 seconde et 30 secondes, ou entre 1,44 seconde et 9 secondes, ou entre 1,8 seconde et 6 secondes, ou entre 2,4 secondes et 4,5 secondes. Cette fréquence peut être également comprise entre 18 secondes et 30 minutes, préférentiellement entre 24 secondes et 6 minutes, plus préférentiellement entre 36 secondes et 4 minutes, et plus préférentiellement encore entre 72 secondes et 3 minutes. Le nombre de flashs par heure est choisi en fonction de l'intensité et la durée des flashs (voir ci-dessous). En général, l'intensité de la lumière apportée sous forme de flashs est comprise entre 50 et 1000 µmol. m⁻². s⁻¹, de préférence entre 50 et 500 µmol. m⁻². s⁻¹, ou 50 et 400 µmol. m⁻². s⁻¹, et plus préférentiellement entre 150 et 300 µmol. m⁻². s⁻¹. Par définition, 1 µmol. m⁻². s⁻¹ correspond à 1µE m⁻². s⁻¹ (Einstein), unité souvent utilisée dans la littérature.

Selon un mode particulier de l'invention, l'intensité de la lumière est comprise entre 50 et 200 µmol. m⁻². s⁻¹, la période temporelle de la fréquence des flashs est comprise entre 10 secondes et 60 minutes pour une durée de flash comprise entre 1 seconde et 1 minute.

Selon l'invention, quelles que soient les conditions d'éclairement, l'intensité lumineuse apportée aux algues en culture, exprimée en micromoles de photons par seconde par mètre carré (µmol. m⁻². s⁻¹), varie au moins une fois dans une même heure. L'amplitude de cette variation d'intensité de lumière est généralement comprise entre 50 et 1000, ou entre 50 et 800, ou entre 100 et 600 µmol. m⁻². s⁻¹. L'intensité de la lumière peut également varier entre 50 et 400 µmol. m⁻². s⁻¹. De préférence, l'amplitude de la variation d'intensité de lumière est entre 70 et 300 µmol. m⁻². s⁻¹ et plus préférentiellement entre 100 et 200 µmol. m⁻². s⁻¹. En conditions d'éclairement discontinu, ladite intensité lumineuse peut atteindre successivement, plusieurs fois dans l'heure, par exemple, les valeurs 0 et 50 µmol. m⁻². s⁻¹, les valeurs 0 et 100 µmol. m⁻². s⁻¹ ou préférentiellement encore les valeurs 0 et 200 µmol. m⁻². s⁻¹. Elle peut également atteindre successivement, plusieurs fois dans l'heure, par exemple, les valeurs 0 et 300 µmol. m⁻². s⁻¹, les valeurs 0 et 600 µmol. m⁻². s⁻¹, les valeurs 0 et 800 µmol. m⁻². s⁻¹ ou encore les valeurs 0 et 1000 µmol. m⁻². s⁻¹.

Selon un mode de l'invention et quelles que soient les conditions d'éclairement, l'intensité de la lumière apportée à la culture varie en fonction de la densité cellulaire. Plus la culture devient dense, plus la lumière peut être intense. La densité cellulaire est le nombre de cellules par ml et elle est mesurée selon les techniques connues de l'homme de l'art. Quand la culture, au stade final, atteint une densité entre 10⁷ et 10⁸ cellules par ml, l'intensité lumineuse peut être augmentée jusqu'à entre 50 et 400 µmol. m⁻². s⁻¹ par exemple, de préférence, entre 50 et 150 µmol. m⁻². s⁻¹.

Selon certains modes de réalisation, par exemple, quand la durée des flashs est par exemple de moins d'une minute, ou moins d'une seconde, l'intensité de la lumière peut être plus importante par rapport aux valeurs citées ci-dessus. Quand la culture atteint une densité entre 10⁶ et 10⁷ cellules par ml, l'intensité lumineuse peut être augmentée jusqu'à entre 30 et 500 µmol. m⁻². s⁻¹, par exemple, de préférence, entre 50 et 400 µmol. m⁻². s⁻¹. Quand la culture, au stade final, atteint une densité entre 10⁷ et 10⁸ cellules par ml, l'intensité lumineuse peut être augmentée jusqu'à entre 100 et 1000 µmol. m-². s⁻¹ par exemple, de préférence, entre 200 et 500 µmol. m⁻². s⁻¹.

Selon un mode de l'invention, la quantité de lumière apportée à la culture dans l'heure reste entre certaines valeurs. Elle est comprise entre environ 2000 et 600 000, de préférence entre 2000 et 300 000 µmol. m⁻². Elle peut être comprise entre environ 4000 et 200 000 µmol. m⁻², par heure. Selon un mode de l'invention, la culture est illuminée avec 120 flashs par heure, chaque flash ayant une durée de 10 secondes et une intensité de 200 µmol. m⁻². s⁻¹, ce qui donne un apport total de lumière par heure de 240 000 µmol. m⁻².

Comme décrit pour l'intensité lumineuse ci-dessus, et selon un mode de l'invention, la quantité de lumière apportée à la culture par heure peut varier en fonction de la densité cellulaire. Au stade initial de la culture quand la densité cellulaire est 10⁵ et 5 x 10⁵ cellules par ml, l'apport total de lumière dans l'heure est généralement compris entre environ 1500 et 8000, de préférence 1500 et 6000 µmol. m⁻², de préférence encore entre 2000 et 5000 µmol. m⁻². Quand la culture atteint une densité entre 10⁶ et 10⁷ cellules par ml, l'apport total de lumière dans l'heure peut être augmenté jusqu'à entre 6000 et 67 000 µmol. m⁻², de préférence, entre 6000 et 50 000 et de préférence encore entre 12 000 et 45 000 µmol. m⁻², par exemple. Au stade final de la culture, à une densité cellulaire entre 10⁷ et 10⁸ cellules par ml, l'apport total de lumière dans l'heure peut être augmenté jusqu'à entre 45 000 et 300 000, par exemple de préférence, entre 45 000 et 200 000 µmol. m⁻², et par exemple, de préférence encore, entre 50 000 et 150 000 µmol. m⁻². Au stade final de la culture (à une densité cellulaire entre entre 10⁷ et 10⁸ cellules par ml), la culture est illuminée avec 30 flashs par heure, chaque flash ayant une durée de 30 secondes et une intensité entre 50 et 150 µmol. m⁻². s⁻¹, ce qui donne un apport total de lumière par heure de 45 000 à 135 000 µmol. m⁻².

Selon un mode de l'invention, par exemple, quand la durée des flashs est par exemple de moins d'une minute, ou de moins d'une seconde, au stade initial de la culture (à une densité cellulaire entre 10⁵ et 5 x 10⁵ cellules par ml), la culture est illuminée avec 30 flashs par heure, chaque flash ayant une durée de 10 secondes et une intensité entre 50 et 100 µmol. m⁻². s⁻¹, ce qui donne un apport total de lumière par heure de 15 000 µmol. m⁻² à 30 000 µmol. m⁻². Puis au stade intermédiaire (à une densité cellulaire entre 10⁶ et 10⁷ cellules par ml), la culture est illuminée avec 50 flashs par heure, chaque flash ayant une durée de 10 secondes et une intensité entre 200 et 300 µmol. m⁻². s⁻¹, ce qui donne un apport total de lumière par heure de 100 000 à 150 000 µmol. m⁻². Puis, au stade final de la culture (à une densité cellulaire entre entre 10⁷ et 10⁸ cellules par ml), la culture est illuminée avec 120 flashs par heure, chaque flash ayant une durée de 10 secondes et une intensité entre 350 et 450 µmol. m⁻². s⁻¹, ce qui donne un apport total de lumière par heure de 420 000 à 540 000 µmol. m⁻².

L'apport de lumière dans les cultures peut être obtenu par des lampes réparties autour de la paroi externe des fermenteurs. Une horloge déclenche ces lampes pour des temps d'éclairement définis. Les fermenteurs se situent préférentiellement dans une enceinte à l'abri de la lumière du jour, dont on peut contrôler la température ambiante.

Ainsi qu'a pu le constater le déposant, le fait que les souches ainsi sélectionnées présentent de bonnes aptitudes à croître en mode mixotrophe, en présence d'une lumière discontinue et/ou variable, prédispose lesdites souches à une production plus élevée de caroténoïdes, notamment de lutéine.

Le procédé de culture permet ainsi de sélectionner des souches du genre *Scenedesmus,* à caractère mixotrophe, similaires à celle isolée par le demandeur et déposée à la CCAP, sous le numéro CCAP 276/75 et ayant un haut rendement en lutéine. Ce procédé de culture est caractérisé en ce qu'il comprend les étapes suivantes :
a) la culture en mode mixotrophe d'une ou plusieurs souches du genre *Scenedesmus* dans des conditions d'éclairement discontinu et/ou variable au cours du temps, l'éclairement présentant des variations d'intensité dont l'amplitude est comprise entre 5 µmol. m⁻². s⁻¹ et 1000, de préférence entre 5 et 400 µmol. m⁻². s⁻¹, ces variations ayant lieu entre 2 et 3600, de préférence 5-400 fois par heure,
b) une étape de maintien de ladite culture sur plusieurs générations, en présence d'un substrat carboné organique dans le milieu de culture, et éventuellement
c) une étape de récupération des microalgues ainsi cultivées.

Par étape de récupération, on entend plus particulièrement l'isolement de la ou des souches dont le nombre de cellules s'est accru le plus au cours desdites générations.

Pour réaliser la sélection des souches, différentes souches du genre *Scenedesmus* peuvent être cultivées, en parallèle, sur des microplaques dans une même enceinte, avec un suivi précis des conditions et de l'évolution des différentes cultures. Il est ainsi aisé de connaître la réponse des différentes souches à l'éclairement discontinu et/ou variable et, le cas échéant, à l'adjonction d'un ou plusieurs substrats carbonés dans le milieu de culture. Les souches qui répondent favorablement à l'éclairement discontinu et/ou variable et aux substrats carbonés offrent généralement un meilleur rendement pour la production de lipides sur le plan qualitatif (la lutéine est plus abondante dans le profil lipidique) et quantitatif (les lipides contiennent une proportion plus élevée de lutéine).

Les microalgues peuvent être sélectionnées dans un fermenteur à partir d'une population hétérogène et dont on cherche à sélectionner les variants avantagés par le mode de sélection selon l'invention, alliant lumière discontinue et/ou variable présentant une gamme d'intensité lumineuse et une fréquence spécifiques, avec des conditions de culture mixotrophes. Dans ce cas, la culture est pratiquée en maintenant les microalgues en cultures sur de nombreuses générations, puis un isolement des composantes devenues majoritaires dans le milieu de culture est effectué au terme de la culture.

Le procédé de culture selon l'invention permet également de produire de la lutéine.

Dans ce cas, le procédé selon l'invention comporte en outre les étapes suivantes :
d) une étape de récupération de la matière hydrophobe, et éventuellement
e) l'extraction de la lutéine de la matière hydrophobe récupérée.

Le procédé de culture selon l'invention peut s'appliquer également à toute espèce du genre *Scenedesmus,* capable de croître dans les conditions mixotrophes selon l'invention, et capable de produire de la lutéine.

Le procédé de culture selon l'invention permet d'optimiser la production de la biomasse obtenue de la culture. Il permet également d'enrichir les microalgues ainsi cultivées en caroténoïdes, notamment en lutéine.

L'invention a donc également pour but l'optimisation de la production de biomasse, ainsi que la production de caroténoïdes, notamment de la lutéine, via la culture de microalgues du genre *Scenedesmus* à caractère mixotrophe, de préférence cultivées ou sélectionnées selon les procédés visés précédemment, puis la récupération des microalgues ainsi cultivées pour en extraire le contenu hydrophobe, en particulier la lutéine. Les souches de l'espèce *Scenedesmus* sp. sont spécialement concernées.

Les méthodes d'extraction et d'analyse des caroténoïdes, dont la lutéine, sont connues de l'homme du métier et sont, par exemple, décrites par S.W. Wright et al., [Wright, S.W. et al.(1991): Improved HPLC method for the analysis of chlorophylls and carotenoids from marine phytoplankton. Marine ecology progress series: Vol. 77 : 183-196)].

L'invention porte également sur les microalgues du genre *Scenedesmus,* susceptibles d'être obtenues selon le procédé de l'invention tel que précédemment décrit. Ces microalgues sont enrichies en lutéine. La matière hydrophobe de telles microalgues comprend généralement plus de 10 %, plus de 25%, ou encore plus de 50% de la lutéine par rapport au pourcentage total de la matière hydrophobe.

### Exemple 1

Les cultures *Scenedesmus* sp. FCC 174 sont réalisées dans des fermenteurs (bioréacteurs) de 2L utiles avec automates dédiés et supervision par station informatique. Le système est régulé en pH via l'ajout de base (solution d'hydroxyde de sodium à 1N) et/ou d'acide (solution d'acide sulfurique à 1N). La température de culture est fixée à 25°C. L'agitation est réalisée grâce à 2 mobiles d'agitation placés sur l'arbre selon la configuration suivante : hélice de Rushton et hélices tripales à pompage descendant. La vitesse d'agitation et le débit d'aération sont régulés au mini = 200 rpm et au maxi = 600 rpm et Qmini =0,5 vvm / Qmaxi = 2 vvm respectivement. Le bioréacteur est équipé d'un système luminaire externe entourant la cuve transparente. L'intensité ainsi que les cycles de lumière sont contrôlés par un automate dédié et supervisé par station informatique.

Les réacteurs sont inoculés avec une pré-culture réalisée sur table d'agitation (140 rpm) en enceinte thermostatée (25°C) et éclairée entre 80 et 100 uE. Les pré-cultures et cultures en bioréacteurs sont réalisées dans le milieu BG11 enrichi en azote [Rippka et al. (1979)]. Le substrat carboné organique utilisé pour la culture en mixotrophie en bioréacteur est du glucose à des concentrations entre 100 mM et 150 mM.

### Suivi des cultures:

La concentration en biomasse totale est suivie par mesure de la masse sèche (filtration sur filtre GFB, Whatman, puis séchage en étuve sous vide, à 65°C et -0,8 bar, pendant 24h minimum avant pesée).

Concernant la quantification des lipides totaux, 10⁸ cellules/mL ont été extraites. Les méthodes d'extraction des lipides sont connues de l'homme du métier et sont, par exemple, décrites par [Bligh, E.G. et Dyer, W.J. (1959) ; A rapid method of total lipid extraction and purification, Can J.Biochem. Physiol 37:911-917].

### Eclairement :

La culture est illuminée avec 120 flashs par heure, chaque flash ayant une durée de 10 secondes et une intensité de 200 µmol. m⁻². s⁻¹.

L'apport de lumière dans les cultures en bioréacteur a été obtenu par des lampes LED (Diodes Electro Luminescentes) réparties autour de la paroi externe du fermenteur. Une horloge déclenche ces LED pour des temps d'éclairement ou des pulses.

### Résultats (n=3) :

| | Masse Sèche (g/L) | Lipides totaux (% de la MS) | Lutéine (mg/g de MS) |
|---|---|---|---|
| Mixotrophie Flash | 38 +/- 1,1 | 11,3 +/- 1,2 | 9,2 +/- 0,1 |
| Hétérotrophie | 36,1 +/-1,5 | 10,5 +/-0,9 | 1,5 +/- 0,1 |

## Revendications

1. Procédé comprenant les étapes suivantes :
a) la culture en mode mixotrophe d'une ou plusieurs souches du genre *Scenedesmus* dans des conditions d'éclairement discontinu et/ou variable au cours du temps, l'éclairement présentant des variations d'intensité entre les phases éclairées et les phases obscures dont l'amplitude est comprise entre 50 µmol. m⁻². s⁻¹ et 1000 µmol. m⁻². s⁻¹ ces variations ayant lieu entre 2 et 3600 fois par heure.
b) une étape de maintien de ladite culture sur plusieurs générations en présence d'un substrat carboné organique dans le milieu de culture, et
c) une étape de récupération des microalgues ainsi cultivées.

2. Procédé selon la revendication 1, **caractérisé en ce que** l'éclairement présente des variations d'intensité dont l'amplitude est comprise entre 50 µmol. m⁻². s⁻¹ et 400 µmol. m⁻².s⁻¹, ces variations ayant lieu entre 2 et 200 fois par heure.

3. Procédé selon la revendication 1 ou 2, **caractérisé en ce que** la microalgue est de l'espèce *Scenedesmus* sp.

4. Procédé selon la revendication 1, 2 ou 3 , **caractérisé en ce que** la culture s'effectue en présence d'un substrat carboné organique à une concentration de 5 mM à 1 M, de préférence de 50 mM à 800 mM, plus préférentiellement de 70 mM à 600 mM, et encore plus préférentiellement de 100 mM à 500 mM, le substrat carboné organique étant choisi parmi l'amidon, le lactate, le lactose, le saccharose, l'acétate, le glycérol, le glucose et les dérivés de cellulose et un mélange de ces molécules.

5. Procédé selon la revendication 4, **caractérisé en ce que** ledit substrat carboné organique présent dans le milieu de culture comprend au moins 5 mM de glucose.

6. Procédé selon la revendication 5, **caractérisé en ce que** l'amplitude des variations d'intensité est comprise entre 50 et 1000, de préférence entre 50 et 400 µmol. m⁻². s⁻¹, de préférence encore entre 70 et 300 µmol. m⁻². s⁻¹, et plus préférentiellement encore entre 100 et 200 µmol. m⁻². s⁻¹.

7. Procédé selon l'une quelconque des revendications 1 à 6, **caractérisé en ce que** l'apport de lumière s'effectue sous forme de flashs.

8. Procédé selon la revendication 7, **caractérisé en ce qu'**un flash a une durée comprise entre 1/10 seconde et 10 minutes, de préférence entre 5 secondes et 10 minutes, de préférence encore entre 10 secondes et 2 minutes, plus préférentiellement encore entre 20 secondes et 1 minute.

9. Procédé selon la revendication 7 ou la revendication 8, **caractérisé en ce que** le nombre de flashs est entre 5 et 3600, de préférence entre 10 et 150, préférentiellement entre 15 et 100, et plus préférentiellement entre 20 et 50 fois par heure.

10. Procédé selon l'une quelconque des revendications 1 à 9, **caractérisé en ce que** l'apport total de lumière par heure en micromoles des photons est entre 2000 à 600 000, de préférence 2000 à 200 000 µmol. m⁻².

11. Procédé selon l'une quelconque des revendications 1 à 10, **caractérisé en ce qu'**il comprend en outre les étapes suivantes :
d) une étape de récupération de la matière hydrophobe, et éventuellement
e) l'extraction de la lutéine de la matière hydrophobe récupérée.

12. Procédé selon l'une quelconque des revendications 1 à 11, **caractérisé en ce que** ladite microalgue du genre *Scenedesmus* correspond à la souche FCC 174, déposée le 13 mars 2012 auprès de la CCAP (Culture Collection of Algae and Protozoa), sous le numéro CCAP 276/75.

13. Biomasse de microalgues du genre *Scenedesmus* capable de produire de la lutéine susceptible d'être obtenue par le procédé de l'une quelconque des revendications 1 à 12, **caractérisée en ce qu'**elle comprend plus de 1% en poids de lutéine par rapport au poids de la matière sèche.

## Patentansprüche

1. Verfahren, umfassend die folgenden Schritte:
a) die Kultur im mixotrophen Modus von einem oder mehreren Stämmen der Gattung *Scenedesmus* unter diskontinuierlichen und/oder variablen Beleuchtungsbedingungen im Laufe der Zeit, wobei die Beleuchtung Lichtstärkeschwankungen zwischen den hellen Phasen und den dunklen Phasen aufweist, deren Amplitude zwischen 50 umol.m⁻².s⁻¹ und 1000 µmol.m⁻².s⁻¹ inbegriffen beträgt, wobei diese Schwankungen zwischen 2 und 3600 Mal pro Stunde stattfinden,
b) einen Schritt des Haltens der Kultur über mehrere Generationen bei Anwesenheit eines organischen kohlenstoffhaltigen Substrats in dem Kulturmilieu, und
c) einen Schritt der Rückgewinnung der derart kultivierten Mikroalgen.

2. Verfahren nach Anspruch 1, **dadurch gekennzeichnet, dass** die Beleuchtung Intensitätsschwankungen aufweist, deren Amplitude zwischen 50 umol.m⁻².s⁻¹ und 400 umol.m⁻².s⁻¹ inklusive beträgt, wobei diese Schwankungen zwischen 2 und 200 Mal pro Stunde stattfinden.

3. Verfahren nach Anspruch 1 oder 2, **dadurch gekennzeichnet, dass** die Mikroalge die Spezies *Scenedesmus* sp. ist.

4. Verfahren nach Anspruch 1, 2 oder 3, **dadurch gekennzeichnet, dass** die Kultur bei Anwesenheit eines organischen kohlenstoffhaltigen Substrats in einer Konzentration von 5 mM bis 1 M, vorzugsweise von 50 mM bis 800 mM, vorzugsweiser von 70 mM bis 600 mM und noch vorzugsweiser von 100 mM bis 500 mM erfolgt, wobei das organische kohlenstoffhaltige Substrat aus der Stärke, dem Lactat, der Lactose, der Saccharose, dem Acetat, dem Glycerol, der Glucose und den Zellulosederivaten und einem Gemisch dieser Moleküle ausgewählt ist.

5. Verfahren nach Anspruch 4, **dadurch gekennzeichnet, dass** das im Kulturmilieu vorhandene organische kohlenstoffhaltige Substrat mindestens 5 mM Glucose umfasst.

6. Verfahren nach Anspruch 5, **dadurch gekennzeichnet, dass** die Amplitude der Intensitätsschwankungen zwischen 50 und 1000, vorzugsweise zwischen 50 und 400 umol.m⁻².s⁻¹ und vorzugsweise auch zwischen 70 und 300 µmol.m⁻².s⁻¹ und noch vorzugsweise zwischen 100 und 200 µmol.m⁻².s⁻¹ inklusive beträgt.

7. Verfahren nach einem der Ansprüche 1 bis 6, **dadurch gekennzeichnet, dass** die Zufuhr von Licht in Form von Blitzen erfolgt.

8. Verfahren nach Anspruch 7, **dadurch gekennzeichnet, dass** ein Blitz eine Dauer zwischen 1/10 Sekunde und 10 Minuten, vorzugsweise zwischen 5 Sekunden und 10 Minuten, vorzugsweise auch zwischen 10 Sekunden und 2 Minuten, noch vorzugsweiser zwischen 20 Sekunden und 1 Minute hat.

9. Verfahren nach Anspruch 7 oder Anspruch 8, **dadurch gekennzeichnet, dass** die Anzahl von Blitzen zwischen 5 und 3600, vorzugsweise zwischen 10 und 150, vorzugsweise zwischen 15 und 100 und noch vorzugsweiser zwischen 20 und 50 Mal pro Stunde beträgt.

10. Verfahren nach einem der Ansprüche 1 bis 9, **dadurch gekennzeichnet, dass** die Gesamtzufuhr von Licht pro Stunde in Photonen-Mikromol zwischen 2000 bis 600000, vorzugsweise 2000 bis 200000 µmol.m⁻², beträgt.

11. Verfahren nach einem der Ansprüche 1 bis 10, **dadurch gekennzeichnet, dass** es ferner die folgenden Schritte umfasst:
d) einen Schritt der Rückgewinnung des hydrophoben Materials, und eventuell
e) die Extraktion von Lutein aus dem rückgewonnenen hydrophoben Material.

12. Verfahren nach einem der Ansprüche 1 bis 11, **dadurch gekennzeichnet, dass** die Mikroalge der Gattung *Scenedesmus* dem Stamm FCC 174 entspricht, hinterlegt am 13. März 2012 bei der CCAP (Culture Collection of Algae and Protozoa) unter der Nummer CCAP 276/75.

13. Biomasse von Mikroalgen der Gattung *Scenedesmus,* die imstande ist, Lutein zu produzieren, das anhand des Verfahrens nach einem der Ansprüche 1 bis 12 gewinnbar ist, **dadurch gekennzeichnet, dass** sie über 1 Gew.-% Lutein in Bezug zum Gewicht der Trockenmasse umfasst.

## Claims

1. A method comprising the following steps:
a) culture, in mixotrophic mode, of one or more strains of the genus *Scenedesmus* under conditions of illumination that is discontinuous and/or variable over time, the illumination having variations in intensity between the illuminated phases and the phases of darkness, the amplitude of which is comprised between 50 µmol ·m⁻² ·s⁻¹ and 1,000 µmol ·m⁻² ·s⁻¹, these variations taking place between 2 and 3,600 times per hour,
b) a step of maintaining said culture over several generations in the presence of an organic carbon-containing substrate in the culture medium, and
c) a step of recovery of the thus cultured microalgae.

2. The method according to claim 1, **characterized in that** the illumination has variations in intensity, the amplitude of which is comprised between 50 µmol·m⁻²·s⁻¹ and 400 µmol·m⁻²·s⁻¹, these variations taking place between 2 and 200 times per hour.

3. The method according to claim 1 or 2, **characterized in that** the microalga is from the species *Scenedesmus* sp.

4. The method according to claim 1, 2 or 3, **characterized in that** the culture is carried out in the presence of an organic carbon-containing substrate at a concentration from 5 mM to 1 M, preferably from 50 mM to 800 mM, more preferentially from 70 mM to 600 mM, and even more preferentially from 100 mM to 500 mM, the organic carbon-containing substrate being selected from starch, lactate, lactose, sucrose, acetate, glycerol, glucose and cellulose derivatives and a mixture of these molecules.

5. The method according to claim 4, **characterized in that** said organic carbon-containing substrate present in the culture medium comprises at least 5 mM of glucose.

6. The method according to claim 5, **characterized in that** the amplitude of the variations in intensity is comprised between 5 and 1,000, preferably between 50 and 400 µmol·m⁻²·s⁻¹, · s⁻¹, yet more preferably between 70 and 300 µmol·m⁻²·s⁻¹ and even more preferentially between 100 and 200 µmol·m⁻²·s⁻¹.

7. The method according to any one of claims 1 to 6, **characterized in that** the light supply is in the form of flashes.

8. The method according to claim 7, **characterized in that** a flash has a duration comprised between 1/10 second and 10 minutes, preferably between 5 seconds and 10 minutes, more preferably between 10 seconds and 2 minutes, even more preferentially between 20 seconds and 1 minute.

9. The method according to claim 7 or claim 8, **characterized in that** the number of flashes is between 5 and 3,600, preferably between 10 and 150, preferentially between 15 and 100, and more preferentially between 20 and 50 times per hour.

10. The method according to any one of claims 1 to 9, **characterized in that** the total light supply per hour in micromoles of photons is between 2,000 to 600,000, preferably between 2,000 to 200,000 µmol·m⁻².

11. The method according to any one of claims 1 to 10, **characterized in that** it further comprises the following steps:
d) a step of recovery of the hydrophobic matter, and optionally
e) the extraction of the lutein from the hydrophobic matter recovered.

12. The method according to any one of claims 1 to 11, **characterized in that** said microalga of the genus *Scenedesmus* corresponds to the strain FCC 174, deposited on March 13, 2012 with the CCAP (Culture Collection of Algae and Protozoa), under the number CCAP 276/75.

13. A biomass of microalgae of the genus *Scenedesmus* capable of producing lutein capable of being obtained by the method of any one of claim 1 to 12, **characterized in that** it comprises more than 1% by weight of lutein with respect to the weight of dry matter.
